# EUROPEAN PATENT SPECIFICATION

(11) **EP 1 755 544 B1**
(45) Date of publication and mention of the grant of the patent: **06.04.2011**
(21) Application number: 05745052.0
(22) Date of filing: 03.05.2005
(51) Int. Cl.: A61K 8/26, A61K 8/28, A61Q 15/00

(54) **ANTIPERSPIRANT OR DEODORANT COMPOSITIONS**
ANTITRANSPIRANT- ODER DEODORANTZUSAMMENSETZUNGEN
COMPOSITIONS ANTI-TRANSPIRANTES OU DEODORANTES

(30) Priority: 13.05.2004 GB 0410616
(43) Date of publication of application: 28.02.2007
(73) Proprietor: Unilever PLC, London EC4Y 0DY (GB); Unilever N.V., 3013 AL Rotterdam (NL)
(72) Inventor: BATISTA, Andrea Paula, Seacroft Yorkshire LS14 2AR (GB)
(74) Representative: Whaley, Christopher
(86) International application number: PCT/EP2005/004828
(87) International publication number: WO 2005/112879

(56) References cited:
- WO-A-02/085320
- WO-A-2004/052325
- WO-A-2004/064792
- WO-A-2004/100910
- GB-A- 2 299 506
- US-A- 5 393 518
- US-A- 6 126 928
- US-B1- 6 375 937
- US-B1- 6 403 069

## Description

The present invention relates to antiperspirant or deodorant compositions and more particularly to aqueous compositions and to their preparation.

### Background and Prior Art

Cosmetic, which is understood herein to mean non-therapeutic, antiperspirant or deodorant formulations are commonly available to the public for application topically to the axilla (underarm) and less commonly to feet. Both contact and non-contact applicators are available, and habits differ from one country to another as which is the most popular. One class of applicators that is popular in parts of Europe, South East Asia and Latin America comprises roll-ons in which a roll-ball distributes a mobile liquid from a reservoir by contact with skin.

In the past, a significant fraction of roll-on formulations have employed a solution of an antiperspirant active in aqueous alcohol, typically aqueous ethanol. Whilst many find the cooling sensation from such formulations pleasurable, on account of alcohol evaporation, alcohol can sting, especially if the formulation is applied to broken or cut skin. Accordingly, the industry has sought alternatives which do not require alcohol.

It is often desirable to incorporate a lypophylic organic liquid in antiperspirant or deodorant formulations, for example a fragrance. When the base composition is aqueous, it is convenient to employ a solubilising emulsifier or surfactant to provide composition homogeneity.

Formulations that are intended for skin application via roll-on dispensers tend, in practice, to be significantly more viscous than water. The actual viscosity of the formulation is often designed in conjunction with the gap between the roll ball and its housing so as to prevent an excessive flow of formulation, but at the same time enable sufficient formulation to be dispensed in a few strokes of the ball to control perspiration and/or malodour formation. The viscosity of the formulation can be increased conveniently by employing a thickener. Antiperspirant or deodorant products can often spend several months in the distribution chain or in users' cabinets before or whilst they are being used. Accordingly, it is desirable that the formulations remain stable until they are fully consumed.

One class of thickeners for aqueous solutions comprises a polyquaternium derivative of hydroxycellulose. However, the inventors have found that emulsion formulation that have been thickened with that class of thickener demonstrate signs of instability, which is evidenced by a reduction in the viscosity of the formulation over time. The rate of change is also increased by elevated temperatures which can occur, for example during storage in warehouses, especially during the summer, or if the roll-on dispenser is exposed to strong sunlight. The roll-on bottle itself remains substantially unchanged during storage, so that the gap between the roll ball and its housing remains unchanged, and a reduction in formulation viscosity increases the risk of the formulation flowing out undesirably quickly under gravity when the bottle is inverted (i.e. having the ball below the bottle reservoir). It would desirable to improve the stability of such formulations, which is to say find a means to reduce the risk of such formulations suffering reduced viscosity or to increase the period before significant viscosity reduction occurs or to enable the product to be stored or transported at higher temperatures without significant viscosity reduction occurring.

### Summary of the Invention

According to a first aspect of the present invention, there is provided a formulation in accordance with claim 1 hereinafter.

By virtue of the incorporation of a stabilising amount of propylene glycol, the risk of the thickened compositions suffering reduced viscosity during storage or transportation is lessened or the period before any significant viscosity reduction occurring is increased or the product can be stored or transported at higher temperatures without significant viscosity reduction occurring.

### Detailed Description of the Invention

The instant invention relates to a means for stabilising an aqueous solution of an antiperspirant active containing a solubilised lypophylic liquid and thickened with a polyquaternium derivative of an hydroxycellulose comprising the introduction of an effective amount of propylene glycol. It is effective, i.e. a stabilising amount, if it lessens the extent to which a corresponding propylene-glycol-free composition would suffer from a reduction in viscosity during storage, for example in a test after 4 weeks at 23°C, and particularly lessens the viscosity reduction by at least 5% preferably at least 10% and advantageously at least 20% in the test.

The composition contains at least 1.5% by weight often at least 2% and in at least some preferred compositions at least 2.5% by weight. Stabilisation tends to increase with increasing concentration of propylene glycol, and this propensity is especially noticeable at lower concentrations, such as up to 3 or 3.5% by weight. The concentration of propylene glycol is not more than 5% by weight of the composition. Although the incorporation of propylene glycol improves the stability of the composition, it has unfortunately been found simultaneously to increase negative sensory attributes perceived by users. For example, increased propylene glycol induces a perception of increased stickiness. Consequently, a balance can be struck between the increased stability and increase in stickiness, a window of opportunity in which a valuable increase in stability can be obtained without an excessive impairment of stickiness. The concentration of propylene glycol is consequentially often chosen in the range of from 2 to 5% by weight, and in many preferred compositions from 2 or 2.5 to 3 or 3.5% by weight.

The polyquaternium derivative of hydroxycellulose is commonly a derivative of an hydroxyethylcellulose. Of the various derivatives available listed in the International Cosmetic Ingredient Dictionary and Handbook, 8th Edition published by the CTFA (Cosmetic Toiletry and Fragrance Association) in 2000, including Polyquaternium 4, polyquaternium 10 and polyquaternium 23, the preferred material is polyquaternium 10. The proportion of polyquaternium thickener to employ depends to at least some extent on the viscosity desired in the composition and can also vary according to the grade of the thickener. The concentration of thickener is not greater than 2.5% and conveniently is not greater than 2% by weight. In some embodiments, the thickener concentration is not more than 0.8%. In some other desirable embodiments, the weight proportion of thickener is from 1 to 2%.

It is often desirable to employ the propylene glycol and the thickener in a weight ratio of from 4:1 to 7.5:1, and in some particularly preferred embodiments from 5:1 to 6:1.

The antiperspirant or deodorant compositions herein contain one or more antiperspirant or deodorant actives.

The composition preferably contains an antiperspirant active in an amount of from 5 to 30% and especially from 5 or 10% to 30 of the weight of the composition. In some desirable embodiments, the compositions contain from 10 to 20% by weight antiperspirant active. In other desirable compositions, the proportion of antiperspirant active is from 6 to 10%, and especially when the active is an aluminium zirconium chlorohydrate or an Al/Zr chlorohydrex complex with glycine.

Antiperspirant actives for use herein are often selected from astringent active salts, including in particular aluminium, zirconium and mixed aluminium/zirconium salts, including both inorganic salts, salts with organic anions and complexes. Preferred astringent salts include aluminium, zirconium and aluminium/zirconium halides and halohydrate salts, such as especially a chlorohydrate. Activated aluminium chlorohydrates can be incorporated, if desired, commonly having a higher Band 3 content. Some literature employs alternative terminology, such as basic aluminium chloride for aluminium chlorohydrate, and aluminium chlorhydrex for complexed chlorohydrate. In some suitable actives, the chlorohydrate on introduction is complexed with a glycol, particularly propylene glycol.

Aluminium halohydrates are usually defined by the general formula Al₂(OH)ₓQ_{y}.wH₂O in which Q represents respectively chlorine, bromine or iodine, (and especially chlorine to form a chlorohydrate) x is variable from 2 to 5 and x + y = 6 while WH₂O represents a variable amount of hydration.

Zirconium actives can usually be represented by the empirical general formula: ZrO (OH)_{2n-nz}B_{z}.wH₂O in which z is a variable in the range of from 0.9 to 2.0 so that the value 2n-nz is zero or positive, n is the valency of B, and B is selected from the group consisting of chlorine (to form a chlorohydrate), other halide, sulphamate, sulphate and mixtures thereof. Possible hydration to a variable extent is represented by wH₂O. Preferably, B represents chlorine and the variable z lies in the range from 1.5 to 1.87. In practice, such zirconium salts are usually not employed by themselves, but as a component of a combined aluminium and zirconium-based antiperspirant.

The above aluminium and zirconium salts may have coordinated and/or bound water in various quantities and/or may be present as polymeric species, mixtures or complexes. In particular, zirconium hydroxy salts often represent a range of salts having various amounts of the hydroxy group. Zirconium aluminium chlorohydrate may be particularly preferred.

Antiperspirant complexes based on the above-mentioned astringent aluminium and/or zirconium salts can be employed. The complex often employs a compound with a carboxylate group, and advantageously this is an amino acid. Examples of suitable amino acids include dl-tryptophan, dl-β-phenylalanine, dl-valine, dl-methionine and β-alanine, and preferably glycine which has the formula CH₂(NH₂)COOH.

It is highly desirable to employ complexes of a combination of aluminium chlorohydrates and zirconium chlorohydrates together with amino acids such as glycine, which are disclosed in US-A-3792068 (Luedders et al). Certain of those Al/Zr complexes are commonly called ZAG in the literature. ZAG actives generally contain aluminium, zirconium and chloride with an Al/Zr ratio in a range from 2 to 10, especially 2 to 6, an Al/Cl ratio from 2.1 to 0.9 and a variable amount of glycine. Actives of this preferred type are available from Westwood, from Summit and from Reheis.

The selection of zirconium aluminium salts, including zriconium aluminium complexes, is advantageous in certain embodiments in that a similar level of antiperspirant efficacy can be attained using a lower concentration of antiperspirant active than for aluminium chlorohydrates. The use of less of an ingredient releases formulation space for other constituents and can reduce the likelihood of visible deposits upon axillary application of the composition. None the less, it will also be recognised that the latter antiperspirant salts can be employed in other embodiments that are practically useful.

The antiperspirant salts can be introduced in the form of powders or in the form of pre-formed solutions, commonly in water. Such powders may, if desired, comprise an adduct with propylene glycol, and pre-formed solutions can likewise contain propylene glycol, to provide not more than the desired proportion of propylene glycol in the fully-formed formulation.

Other actives which may be utilised include astringent titanium salts, for example those described in GB 2299506A.

Herein, in calculating the weight of the active antiperspirant salt, any water of hydration is ignored.

The antiperspirant active salts can be incorporated by employment of preformed aqueous solutions obtainable from the producers, with subsequent dilution as needed with water, optionally or alternatively a fraction of the antiperspirant active can be incorporated from a preformed solution in propylene glycol or an aqueous /propylene glycol mixture. If desired, solid antiperspirant active can be dissolved in water or the aqueous/propylene glycol mixture.

Some preferred compositions herein contain at least 67.5% by weight water and particularly in certain formulations at least 75% by weight water. The weight proportion of water is commonly not more than 87.5% and in many embodiments is up to 85% by weight.

The combined weight of water and antiperspirant active is desirably selected in the range of from 85 to 95% by weight and in a number of highly desirable compositions is at least 88% by weight. In such or other formulations, such combined weight is conveniently up to 93%.

The compositions herein advantageously contain a fragrance material in the form of a lypophylic organic material. Such materials provide a characteristic perfume to the composition, but lypophylic materials are not readily dissolved in the aqueous antiperspirant solution. The fragrance is commonly employed at a concentration of from 0.25 to 2.5% by weight of the composition and in many instances in an amount of at least 0.5%. The proportion is sometimes not greater than 1.5% by weight.

The fragrance, and/or any other lypophylic liquid can be solubilised by employment of a solubilising emulsifier or surfactant. Such solubilisers are often selected from nonionic surfactants, and particularly from polyalkylene oxide-containing nonionic surfactants. Desirable emulsifiers include polyethylene oxide derivatives containing from 10 to 100 and especially from 15 to 50 PEG units. The surfactants can comprise as hydrophobic moiety one or more long chain hydrocarbon or hydrofluorocarbon groups, conveniently containing at least one chain of at least 10 carbons and often from 12 to 25 carbons. Suitable hydrophobic moieties include cetyl, palmityl, stearyl, oleyl, and behenyl. The hydrophobic moiety can comprise a simple entity as exemplified hereinbefore or can comprise a more complex entity such as a derivative of a hydrocarbon chain that is substituted by an hydroxyl group as in derivatives of castor oil. The hydrophobic moiety can be linked directly to the hydrophylic polyalkylene oxide moiety via an ester or in many instances via an ether linkage. The emulsifier can be derived from a polyol such as sorbitol or glycerol, if desired. Illustrative emulsifiers comprise ceteth-20, ceteth-25, ceteth-30, ceteth-35, ceteth-40, glycerol, if desired. Illustrative emulsifiers comprise ceteth-20, ceteth-25, ceteth-30, ceteth-35, ceteth-40, steareth-20, steareth-25, steareth-30, steareth-35, steareth-40, ceteareth-20, ceteareth-25, ceteareth-30, ceteareth-35, ceteareth-40. Preferred solubilisers include PEG derivatives of castor oil. A mixture of emulsifiers can be employed, such as a mixture of the castor oil derivative with a steareth or ceteareth emulsifier, for example in a weight ratio of thrice the weight of the castor oil-based solubiliser.

The proportion of the solubiliser to employ is often selected in the range of from 1.5 to 8% of the composition, and in a number of instances from 1.5 to 5% by weight. Desirably, the solubiliser is employed at a weight ratio to lypophylic oil in the range of from 1:1 to 6:1 and particularly from 1:1 to 4:1.

The compositions herein can additionally comprise an amino preservative. Materials such as particularly an alkanolamine or an aminopolycarboxylic acid/salt chelating agent act as preservatives during storage whilst providing functional benefits on topical application. Trialkanolamines are especially desirable, for example triethanolamine. The aminopolycarboxylic acids such as EDTA (ethylene diaminotetramethylenecarboxylic acid) or DTPA (diethylenetriaminopentamethylenecarboxylic acid) or their water-soluble salts are suitable alternatives. The proportion of the amino preservative is often up to 1.0% by weight, for example from 0.25 to 0.75% by weight chelating preservatives.

The formulations herein can comprise one or more deodorant actives in addition to or instead of the aforementioned chelating preservatives, including chlorinated aromatics, of which materials known as triclosan, and chlorhexidine warrant specific mention. Yet another class of suitable deodorant actives comprises polymeric biguanide salts such as available under the trademark Cosmosil™. These two classes may be employed in amounts selected in the range of from 0.001 to 1%, and particularly from 0.1 to 0.5% by weight. Yet other suitable deodorant actives include zinc salts such as zinc oxide, hydroxide, carbonate, phenol sulphonate or ricinoleate and particular salts that are soluble in the formulation.

In some especially chosen embodiments of the present invention, the compositions comprise one or more of the following:-
from 80 to 95% by weight together of water plus an aluminium or aluminium/zirconium astringent antiperspirant salt, preferably containing from 67.5 to 80% by weight of water plus 15 to 25% of the astringent antiperspirant salt; preferably selected from an aluminium chlorohydrate, an aluminium zirconium chlorohydrate and an aluminium zirconium chlorohydrate complex with glycine; or from 75 to 85% or 87.5% by weight of water and from 6 to 10% by weight of an aluminium zirconium chlorohydrate and an aluminium zirconium chlorohydrate complex with glycine
from 0.4 to 0.8% by weight of a polyquaternium derivative of a hydroxycellulose, preferably polyquaternium-10;
from 0.5 to 2% by weight of a lypophylic oil, preferably a fragrance oil;
oil solubiliser and emulsifier, preferably from 3 to 6% by weight, advantageously comprising an ethoxylated nonionic emulsifier and especially containing at least 1% by weight of an ethoxylated castor oil optionally hydrogenated optionally together with a further ethoxylated nonionic emulsifier
from 2 to 5% by weight of propylene glycol and optionally up to 1% by weight of an aminoalkanol, preferably triethanolamine, or an aminopolycarboxylic acid or salt, preferably EDTA or DTPA.

Formulations according to the invention and especially those of the aforementioned especially chosen embodiments have the advantage of being translucent, and often sufficiently so to be transparent, whilst at the same time demonstrating significantly improved stability against thinning without incurring excessive sensory negative attributes, such as stickiness, and without being based on ethanol.

Compositions according to the present invention can be made by mixing the ingredients together in the desired proportions in a suitable mixing vessel. Conventional low shear mixers can be employed. Advantageously, the process can be conducted at or about ambient temperature, by which is contemplated a temperature of from about 15 to 30°C or at an elevated temperature, such as up to 70°C. Although the order of introduction of the ingredients is at the convenience of the producer, it is often of practical advantage to introduce water or an aqueous solution into the production vessel first and thereafter introduce remaining ingredients into a bulk material. One convenient method comprises hydrating the thickener at an elevated temperature of from for example 50 to 70°C, and thereafter introducing the remaining constituents, possibly without continued heating. It is often convenient to premix the fragrance with the solubiliser and to introduce the resultant mixture last, preferably when the temperature of the remainder of the composition is or has fallen to below 40°C.

Advantageously, by dissolving the various constituents of the invention compositions to form homogenous mixtures, translucent and comparatively stable formulations are obtained.

Compositions according to the present invention are particularly suitable for application to human skin in a non-therapeutic method of controlling perspiration or odour employing a roll-on or similar dispenser. Such dispensers include those described WO 00/64302 WO 00/49908 or US 6511243.

In a further aspect of the present invention there is provided a non-therapeutic method for controlling perspiration or odour by applying topically to human skin a composition in accordance with the first aspect of the invention, and especially to armpits.

Having described the invention in general terms and preferred embodiments thereof, specific embodiments are now given in more detail by way of example only.

**Table 1**

| Materials | | | |
|---|---|---|---|
| INCI name | Trade Name | Supplier | Function |
| Polyquaternium 10 Sorbeth-30 | Ucare JR30M Atlas G-2330 | Dow Uniqema | Thickener emulsifier |
| PEG-40 hydrogenated castor oil | Cremophor RH40 | BASF | Solubiliser |
| Triethanolamine | | BASF | preservative |
| Aluminium Zirconium tetrachlorhydrex Gly | Rezal 36GC | Reheis | antiperspirant astringent active salt |

### Examples 1 and 2 and comparison A

This comparison and Examples demonstrate stability improvement. The compositions in accordance with the summary in the Table 2 below were prepared by introducing water into a mixing vessel at laboratory ambient temperature water, heating it to about 60°C, introducing the thickener and maintaining the temperature and stirring until the thickener was hydrated. Then, the stabiliser, emulsifier and amine were introduced, and followed by the antiperspirant active salt and stirring was continued until dissolution was complete. Thereafter, the remaining ingredients were introduced and mixing continued until the composition had an homogenous appearance. Finally, when the composition temperature had fallen below 40°C, the fragrance which had been pre-mixed with the solubiliser was introduced with stirring. The compositions were then poured into dispensers described in WO 00/64302, the cap fitted and the bottles were stored in the dark in a cabinet maintained at 45°C. The viscosity of the compositions was tested at laboratory ambient temperature (about 23°C) both prior to storage and at intervals during storage in order to identify the extent to which the composition resists thinning and periodically inspected to determine whether the compositions had altered their appearance.

**Table 2**

| | Comp A | Ex 1 | Ex 2 |
|---|---|---|---|
| | % by weight | | |
| Water | 78.5 | 75.5 | 74.0 |
| antiperspirant active | 17.4 | 17.4 | 17.4 |
| thickener | 0.6 | 0.6 | 0.6 |
| propylene glycol | 0 | 3.0 | 5.0 |
| triethanolamine | 0.5 | 0.5 | |
| Emulsifier | 3.0 | 3.00 | 3.0 |

| Stability data | | | |
|---|---|---|---|
| % reduction in viscosity (4 weeks) | 95 | 34 | 33 |

Comparison A and Examples 1 and 2 were all translucent and had desirable sensory properties, for example as regards their stickiness on topical application, and the presence of little or no visible deposits. The three formulations had an initial viscosity in the region of 740 to 800 mPa.s However, Examples 1 and 2 demonstrated substantially increased resistance to a loss of viscosity during storage, a reduction of about 1/3^{rd} of the initial viscosity compared with Comp A which lost virtually all its initial viscosity. Thus, Examples 1 and 2 exemplify a window within which stability is significantly improved without significantly impairing sensory properties.

Furthermore, when the proportion of propylene glycol in the formulation was increased to 10% by weight, with a corresponding reduction of water content, the resultant formulation exhibit impaired sensory properties and in particular significantly greater stickiness.

### Examples 3 to 9

These Example compositions were made in the same way as Example 1.

**Table 3**

| | Ex 3 | Ex 4 | Ex 5 | Ex 6 | Ex 7 | Ex 8 | Ex 9 |
|---|---|---|---|---|---|---|---|
| Ingredient | % by weight | | | | | | |
| Water | 73.0 | 76.1 | 73.5 | 72.5 | 82.5 | 77.3 | 69.0 |
| antiperspirant active | 17.4 | 17.4 | 17.4 | 17.4 | 8.0 | 10.0 | 21.0 |
| thickener | 0.6 | 0.5 | 0.6 | 0.6 | 0.6 | 0.7 | 0.8 |
| propylene glycol | 3.0 | 3.0 | 3.0 | 3.0 | 3.0 | 3.5 | 2.7 |
| Fragrance | 1.2 | 1.2 | 0.6 | 1.2 | 1.2 | 1.2 | 1.2 |
| triethanolamine | | | 0.5 | 0.5 | 0.5 | 0.5 | 0.5 |
| Emulsifier | 3.0 | | 3.0 | 3.0 | 3.0 | 3.0 | 3.0 |
| Solubiliser | 1.8 | 1.8 | 1.4 | 1.8 | 1.8 | 1.8 | 1.8 |

The compositions of Examples 3 to 9 all enjoyed a combination of acceptable sensory properties, translucency and stability.

Compositions having similar stability and sensory properties to the corresponding compositions are obtained by introducing aluminium chlorohydrate (ACH-303 [50% aqueous solution, Summit]) or aluminium zirconium chlorohydrate (Zirconal-50 [50% aqueous solution, B K Giulini]) instead of the antiperspirant active of Examples 1 to 9.

## Claims

1. An antiperspirant or deodorant formulation comprising a lypophilic organic liquid,
an emulsifier to solubilise the organic liquid an aqueous solution of an antiperspirant active,
a thickener and propylene glycol
**characterised in that** the composition comprises at least 65% by weight of water and from 5 to 30% by weight of the antiperspirant active, from 0.5 to 2.5% by weight of a thickener which is a polyquaternium derivative of an hydroxycellulose and the propylene glycol is present in a stabilising amount at a concentration of from 1.5 to 5% by weight.

2. A composition according to claim 1 in which the amount of propylene glycol is at least 2% by weight.

3. A composition according to claim 2 in which the amount of propylene glycol is from 2 to 3.5% by weight.

4. A composition according to any preceding claim in which the polyquaternium thickener is polyquaternium 10.

5. A composition according to any preceding claim in which the antiperspirant active is selected from an aluminium chlorohydrate, an aluminium zirconium chlorohydrate or a complex of either with glycine.

6. A composition according to any preceding claim in which the antiperspirant active is present in a concentration of from 15 to 25% by weight.

7. A composition according to claim 5 or 6 in which the Antiperspirant is an aluminium zirconium chlorohydrate, optionally complexed with glycine, present at a concentration of from 6 to 10% by weight.

8. A composition according to any preceding claim in which the lypophylic organic liquid comprises fragrance material.

9. A composition according to any preceding claim in which the emulsifier is present in an amount of from 1.5 to 8% by weight.

10. A composition according to any preceding claim in which the lypophylic organic liquid and emulsifier are present in a weight ratio of from 0.5-2 to 1.5-8.

11. A composition according to any preceding claim in which the emulsifier comprises a nonionic ethoxylated ether.

12. A composition according to claim 11 in which the emulsifier comprises an ethoxylated castor oil, optionally hydrogenated.

13. A non-therapeutic method of reducing perspiration or controlling odour comprising applying topically to skin a composition according to any preceding claim.

## Patentansprüche

1. Transpirationshemmende oder desodorierende Formulierung, umfassend eine IIpophile organische Flüssigkeit,
einen Emulgator, um die organische Flüssigkeit zu solubilisieren,
eine wässrige Lösung eines transpirationshemmenden Wirkstoffs,
ein Verdickungsmittel und Propylenglykol,
**dadurch gekennzeichnet, dass** die Zusammensetzung wenigstens 65 Gewichts-% Wasser und 5 bis 30 Gewichts-% des transpirationshemmenden Wirkstoffs, 0,5 bis 2,5 Gewlchts-% eines Verdickungsmittels, das ein Polyquaternium-Derivat einer Hydroxycellulose ist, umfasst und Propylenglykol in einer stabilisierenden Menge mit einer Konzentration von 1,5 bis 5 Gewichts-% vorhanden ist.

2. Zusammensetzung gemäß Anspruch 1, wobei die Menge an Propylenglykol wenigstens 2 Gewichts-% ist.

3. Zusammensetzung gemäß Anspruch 2, wobei die Menge an Propylenglykol 2 bis 3,5 Gewichts-% ist.

4. Zusammensetzung gemäß einem vorangehenden Anspruch, wobei das Polyquaternium-Verdickungsmittel Polyquaternium 10 ist.

5. Zusammensetzung gemäß einem vorangehenden Anspruch, wobei der transpirationshemmende Wirkstoff aus einem Aluminiumchlorhydrat, Aluminium-zirkonium-chlorhydrat oder einem Komplex von jedem mit Glycin ausgewählt ist.

6. Zusammensetzung gemäß einem vorangehenden Anspruch, wobei der transpirationshemmende Wirkstoff in einer Konzentration von 15 bis 25 Gewlchts-% vorliegt.

7. Zusammensetzung gemäß Anspruch 5 oder 6, wobei der transpirationshemmende Wirkstoff ein Aluminium-zirkonium-chlorhydrat, gegebenenfalls komplexiert mit Glycin, ist, das In einer Konzentration von 6 bis 10 Gewichts-% vorliegt.

8. Zusammensetzung gemäß einem vorangehenden Anspruch, wobei die lipophile organische Flüssigkeit Duftstoffmaterial umfasst.

9. Zusammensetzung gemäß einem vorangehenden Anspruch, wobei der Emulgator in einer Menge von 1,5 bis 8 Gewichts-% vorliegt.

10. Zusammensetzung gemäß einem vorangehenden Anspruch, wobei die lipophile organische Flüssigkeit und der Emulgator in einem Gewichtsverhältnis von 0,5-2 bis 1,5-8 vorliegen.

11. Zusammensetzung gemäß einem vorangehenden Anspruch, wobei der Emulgator einen nicht-ionischen ethoxylierten Ether umfasst.

12. Zusammensetzung gemäß Anspruch 11, wobei der Emulgator ein ethoxylierte Rizinusöl, das gegebenenfalls hydriert ist, umfasst.

13. Nicht-therapeutisches Verfahren zur Verringerung der Transpiration oder zur Geruchsbekämpfung, umfassend topisches Auftragen einer Zusammensetzung gemäß einem vorangehenden Anspruch auf Haut.

## Revendications

1. Composition anti-transpirante ou déodorante comprenant un liquide organique lipophile, un émulsifiant pour solubiliser le liquide organique, une solution aqueuse d'un actif anti-transpirant, d'un épaississant et de propylène glycol, **caractérisée en ce que** la composition comprend au moins 65 % en poids d'eau et de 5 à 30 % en poids d'actif anti-transpirant, de 0,5 à 2,5 % en poids d'épaississant qui est un dérivé d'hydroxycellulose du type Polyquaternium et le propylène glycol est présent dans une quantité stabilisante à une concentration allant de 1,5 à 5 % en poids.

2. Composition selon la revendication 1, dans laquelle la quantité de propylène glycol est d'au moins 2 % en poids.

3. Composition selon la revendication 2, dans laquelle la quantité de propylène glycol est de 2 à 3,5 % en poids.

4. Composition selon l'une quelconque des revendications précédentes, dans laquelle l'épaississant du type Polyquaternium est le Polyquaternium 10.

5. Composition selon l'une quelconque des revendications précédentes, dans laquelle l'actif anti-transpirant est choisi parmi le chlorhydrate
d'aluminium, le chlorhydrate d'aluminium-zirconium ou un complexe des deux avec de la glycine.

6. Composition selon l'une quelconque des revendications précédentes, dans laquelle l'actif anti-transpirant est présent dans une concentration allant de 15 à 25 % % en poids.

7. Composition selon la revendication 5 ou 6, dans laquelle l'anti-transpirant est le chlorhydrate d'aluminium-zirconium, éventuellement complexé avec de la glycine, présent à une concentration allant de 6 à 10 % en poids.

8. Composition selon l'une quelconque des revendications précédentes, dans laquelle le liquide organique lipophile comprend du parfum.

9. Composition selon l'une quelconque des revendications précédentes, dans laquelle l'émulsifiant est présent dans une quantité allant de 1,5 à 8 % en poids.

10. Composition selon l'une quelconque des revendications précédentes, dans laquelle le liquide organique lipophile et l'émulsifiant sont présents selon un rapport pondéral allant de 0,5-2 à 1,5-8.

11. Composition selon l'une quelconque des revendications précédentes, dans laquelle l'émulsifiant comprend un éther éthoxylé non ionique.

12. Composition selon la revendication 11, dans laquelle l'émulsifiant comprend de l'huile de ricin éthoxylée éventuellement hydrogénée.

13. Procédé non thérapeutique pour réduire la transpiration ou réguler les odeurs comprenant l'application topique cutanée d'une composition selon l'une quelconque des revendications précédentes.
